# EUROPEAN PATENT SPECIFICATION

(11) **EP 3 157 910 B1**
(45) Date of publication and mention of the grant of the patent: **14.03.2018**
(21) Application number: 15730477.5
(22) Date of filing: 17.06.2015
(51) Int. Cl.: C07D 307/42, C07D 307/46

(54) **PROCESS FOR THE SELECTIVE OXIDATION OF 5-HYDROXYMETHYLFURFURAL**
VERFAHREN ZUR SELEKTIVEN OXIDATION VON 5-HYDROXYMETHYLFURFURAL
PROCEDE POUR L'OXYDATION SELECTIVE DE 5-HYDROXYMETHYLFURFURAL

(30) Priority: 17.06.2014 EP 14172678
(43) Date of publication of application: 26.04.2017
(73) Proprietor: Annikki GmbH, 8074 Raaba-Grambach (AT); Microinnova Engineering GmbH, 8412 Allerheiligen bei Wildon (AT)
(72) Inventor: MIHOVILOVIC, Marko, 2380 Perchtoldsdorf (AT); SCHÖN, Michael, 1140 Wien (AT); DOCHEV, Stefan, 1404 Sofia (BG)
(74) Representative: Schwarz & Partner Patentanwälte OG
(86) International application number: PCT/EP2015/063578
(87) International publication number: WO 2015/193364

(56) References cited:
- WO-A2-2008/054804

## Description

The present invention relates to selective oxidation of 5-hydroxymethylfurfural. 5-Hydroxymethyl-2-furfural (HMF) of formula 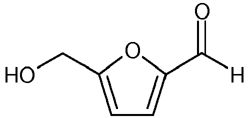 plays an important role in renewable carbohydrate technology and reflects a central intermediate in furan chemistry. Triple carbohydrate monomer dehydration of sugars leads to the formation of HMF, which is widely known in literature. HMF provides three sites of chemical interest - the 5-hydroxymethyl group, the 2-carbaldehyde group and the furan ring itself. By far of highest interest to industry are the two side chains, which can be oxidized to obtain various furan derivatives.

According to the present invention, the four oxidized HMF derivatives 5-hydroxymethylfuran-2-carboxylic acid (HMFCA) of formula 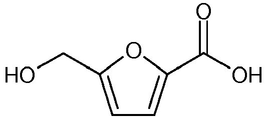 2,5-diformylfuran (DFF) of formula 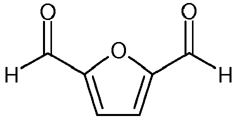 5-formylfuran-2-carboxylic acid (FFCA) of formula 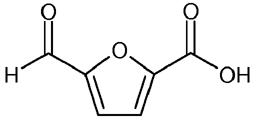 and 2,5-furandicarboxylic acid (FDCA) of formula 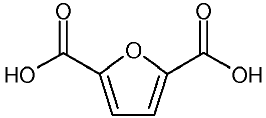 are of particular interest.

HMFCA may be regarded as a result of selective oxidation of the aldehyde group in HMF to obtain the carboxylic acid. For such selective oxidation, only a small number of protocols are known. In most of the cases, expensive silver-based reagents are used in stoichiometric amount to synthesize HMFCA. Silver oxide in basic (NaOH) aqueous medium (Bull. Soc. Chim. Fr. 1987, 5, 855-860) as well as mixed silver-copper catalysts Ag₂O-CuO/O_{2/}NaOH/H₂O (US 3,326,944, 1967) are the most commonly used reagents. Economically, these reagents cannot be applied on large industrial scale. Therefore, precious metal catalysts (especially platinum catalysts) were proposed, e.g. as described in ChemSusChem 2009, 2, 1138-1144; ChemSusChem 2009, 2, 672-675; Catal. Today 2011, 160, 55-60; Green Chem. 2011, 13, 824-827; Green Chem. 2011, 13, 2091-2099) or ruthenium-based catalysis (Top Catal. 2011, 54, 1318-1324; Catal. Lett. 2011, 141, 1752-1760). The oxidation process was mainly carried out in the presence of air and in aqueous reaction environment to synthesize HMFCA in good yield and with high turnover frequency (TOF) rendering the process economically and environmentally benign.

In the synthesis of DFF, a larger number of protocols is known. In batch synthesis, classical oxidation reactions using nitric acid (J. Chem. Soc. Trans. 1912, 101, 1074-1081), lead-(IV)-acetate/pyridine (Tetrahedron 1970, 26, 1291-1301), CrO₃/pyridine or Ac₂O/DMSO (Noguchi Kenkyusho Jiho 1978, 21, 25-33; JP7909260, 1979**;** JP8049368, 1980**),** BaMnO₄/benzene/CCl₄/1,2-dichloroethane (Bull. Soc. Chim. Fr. 1987, 5, 855-860; J. Heterocycl. Chem. 1983, 20, 233-235) or 4-substituted TEMPO/NaOCl/KBr (J. Heterocycl. Chem. 1995, 32, 927-930) are known.

Taking benefit of catalysis, extensive research was already carried out using homogeneous and heterogeneous catalysis. DFF could be synthesized in batch using cobalt, manganese, zinc, cerium or zirconium salts together with a gaseous oxidant (US 2003/055271 A1, 2003**;** Adv. Synth. Catal. 2001, 343, 102-111; WO 01/072732 A2, 2001**;** CA2400165 A1, 2001**;** WO 2010/132740 A2, 2010**;** Catal. Sci. Technol. 2012, 2, 79-81). Furthermore, also diverse vanadium catalysts were reported (ChemSusChem 2011, 4, 51-54; Green Chem. 2011, 13, 554-557; J. Mater. Chem. 2012, 22, 3457-3461). In the heterogeneous catalysis, mainly vanadium- (Pure Appl. Chem. 2012, 84, 765-777; ChemCatChem 2013, 5, 284-293), manganese- (Green Chem. 2012, 14, 2986-2989) and silver-based catalysts (WO 2012/073251 A1, 2012**;** Appl. Catal. B 2014, 147, 293-301) were applied in organic solvents.

Technologically different, also the approaches of sonochemistry (Org. Prep. Proced. Int. 1995, 27, 564-566; Pol. J. Chem. 1994, 68, 693-698) and electrochemistry (Synthesis 1996, 11, 1291-1292) were followed - both of inferior interest for selective, large scale processes on industrial scale.

Although many publications dedicated to the selective oxidation of HMF to DFF are published in literature, only a limited number of described conditions could potentially find industrial application, meeting the requirements for safe, fast, environmentally and economically benign processes. However, reported processes rely on the use of organic solvents, which are troublesome when used in combination with powerful, pressurized oxidants such as pure oxygen. In addition, continuous flow technology was only used so far with a quite specific reaction strategy, wherein a hypervalent iodine species (BAIB) or HNO₃ were used in combination with catalytic amounts of TEMPO (Beilstein J. Org. Chem. 2013, 9, 1437-1442; Green Chem. 2013, 15, 1975-1980).

A further oxidized derivative of HMF is FFCA, which due to its high reactivity and instability is only poorly reported in literature. It can be synthesized using complex catalytic systems such as 4-BzOTEMPO/acetylcholine chloride/Py*HBr₃ in biphasic reaction medium (Bull. Chem. Soc. Jpn. 2009, 82, 1000-1002), strongly acidic conditions under gold catalysis (Catal. Sci. Technol. 2012, 2, 79-81) or precious metal catalysis in flow, but without precise determination of residence times and space-time-yields rendering the process less attractive for cost-efficient production of FFCA (Top Catal. 2010, 53, 1264-1269).

FDCA was also reported as an oxidized furan derivative of particular interest, due to its potential application as replacement for terephthalic acid in polyester synthesis. Also here, classical oxidation was carried out using nitric acid (Chem. Weekblad 1910, 6, 717-727; Noguchi Kenkyusho Jiho 1979, 22, 20-27; Pol. J. Chem. 1994, 68, 693-698) or permanganate (Bull. Soc. Chim. Fr. 1987, 5, 855-860) to selectively give FDCA as product. In the field of catalytic processes, homogeneous catalysts from the cobalt-, manganese-, zinc-, cerium- and zirconium-type are readily known (US 2003/055271 A1, 2003**;** Adv. Synth. Catal. 2001, 343, 102-111; WO 01/72732 A2, CA2400165 A1, 2001**;** WO 2010/132740 A2, 2010**;** US 2009/0156841 A1, 2009**;** WO 2011/043661 A1(A2), 2011**;** Catal. Sci. Technol. 2012, 2, 79-81; WO 2012/161967 A1, WO 2012/161970 A2; US20120302769 A1, 2012**).**

Using heterogeneous catalysis, gold (ChemSusChem 2009, 2, 1138-1144; ChemSusChem 2009, 2, 672-675; Top Catal. 2012, 55, 24-32), ruthenium (Top Catal. 2011, 54, 1318-1324; Catal. Lett. 2011, 141, 1752-1760) as well as platinum catalysts (Top. Catal. 2000, 13, 237-242; US 3,326,944, 1967; Stud. Surf. Sci. Catal. 1990, 55, 147-157; Stud. Surf. Sci. Catal. 1991, 59, 385-394; Top Catal. 2010, 53, 1264-1269) were used, eventually also in flow.

Further processes involving reaction of HMF into oxidation products are known from WO 2012/017052 A1 and WO 2008/054804 A2.

However, summarizing the process parameters and characteristics, no precisely determined, environmentally and economically benign, intrinsically safe and scalable process for the modular synthesis of HMFCA, DFF, FFCA and FDCA has been reported yet.

Now, surprisingly a process for the production of different oxidized 5-hydroxymethylfurfural derivatives, such as 5-hydroxymethylfuran-2-carboxylic acid (HMFCA), 2,5-diformylfuran (DFF), 5-formylfuran-2-carboxylic acid (FFCA) and 2,5-furandicarboxylic acid (FDCA) from HMF in the same reactor setup was found.

In one aspect, the present invention provides a process for the selective production of oxidized furan derivatives starting from 5-hydroxymethyl-2-furfural of formula 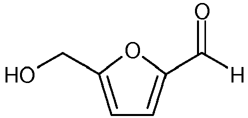 in the presence of a solvent, an oxidation agent, a catalyst, and optionally a base and/or a co-solvent, which is characterized in that
- the oxidation process is carried out continuously in flow,
- there are provided means for varying reaction parameters, such as temperature, pressure, oxidation agent, and/or catalyst.
A process provided by the present invention is also designated herein as "Process(es)" according to the present invention.

Preferably, in the process of the present invention the solvent for the oxidation process is water and a dipolar aprotic solvent is present as a co-solvent. Especially preferably N-methylpyrrolidone is present as a co-solvent.

Oxidized furan derivatives in a process of the present invention comprise at least one aldehyde group and/or at least one carboxylic acid group, preferably 5-hydroxymethylfuran-2-carboxylic acid (HMFCA), 2,5-diformylfuran (DFF), 5-formylfuran-2-carboxylic acid (FFCA) and 2,5-furandicarboxylic acid (FDCA).

A process of the present invention is carried out in a solvent, preferably in water. Optionally a co-solvent may be present. Such co-solvent may be useful for better solubility or enables the use of an enriched HMF stream from previous dehydration reactions as a starting material. Typical examples for co-solvents are dipolar aprotic solvents, such as N,N-dimethylformamide, dimethylsulfoxide, N-methylpyrrolidone; preferably N-methylpyrrolidone.

A process for the production of HMF from carbohydrates, especially fructose, involving the use of NMP as a solvent is disclosed in WO 2014/033289. It has been found that it is possible to perform the process of the present invention using the HMF-enriched product stream, including NMP, of a process as disclosed in WO 2014/033289. Thus, there is no need to remove the NMP contained in said HMF-enriched stream before the oxidation step. Accordingly, in one further preferred embodiment of the present invention, a stream enriched with 5-hydroxymethyl-2-furfural from previous dehydration reactions, in particular dehydrations of sugars, is employed as a starting material. In this embodiment, preferably a stream containing NMP as a solvent is employed and the process does not include a step of removing NMP before the oxidation step.

In this embodiment of the present invention, optionally before the oxidation step one or more pretreatment steps selected from
(i) real stream dilution with water to the desired concentration
(ii) centrifugation in order to separate any black tar formed during the preparation of the stream
(iii) filtration
(iv) passing the solution through a packed-bed cartridge filled with activated charcoal may be carried out.

Furthermore, generally it has been found that dipolar aprotic solvents, including NMP, have advantageous properties especially in the oxidation of HMF to polar products such as FDCA, in terms of the homogenisation of the reaction mixture.

Finally, a positive influence of dipolar aprotic solvents, including NMP, on the stability of the catalysts (protection against deactivation) has been observed,

A process according to the present invention is carried out at a reaction temperature from 50°C to 180°C, preferably from 60°C to 160°C.

In a process of the present invention the reaction temperature for the production of
- 5-hydroxymethylfuran-2-carboxylic acid is from 60°C to 120°C, in particular from 80°C to 120°C, in particular from 100 to 120°C;
- 2,5-diformylfuran is from 100 to 160°C, in particular from 120-160°C, in particular from 140°C to 160°C;
- 5-formylfuran-2-carboxylic acid is from 60°C to 160°C, in particular from 80°C to 140°C, in particular from 100°C to 120°C;
- 2,5-furandicarboxylic acid is from 60°C to 160°C, in particular from 60°C to 120°C, in particular from 80°C to 120°C.

It has been found that when, in the process according to the invention, water is employed as a solvent and NMP is used as a co-solvent, slightly harsher reaction conditions are advantageous, especially in case the desired oxidation product is FDCA. Temperatures ranging from 120°C to 160°C, in particular 140°C to 160°C have been found to be advantageous.

A process according to the present invention is carried out in the presence of an oxidation agent. Such oxidation agent is preferably oxygen or air, in particular compressed oxygen or compressed air.

A process of the present invention is carried out under pressure. A preferred working pressure is from 5 bar to 100 bar, in particular from 10 bar to 80 bar.

In a process according to the present invention, a catalyst is used. Catalysts for the production of oxidation products of HMF are known. A preferred catalyst for the production of DFF in a process of the present invention is K-OMS-2; a preferred catalyst for the production of HMFCA, FFCA and FDCA is 10% Pt/C.

K-OMS-2 and its use in catalysis is known. "OMS-2" stands for cryptomelane type crystalline mixed-valent manganese (oxide)-based octahedral molecular sieve(s). "K in K-OMS-2" stands for potassium. K-OMS-2 has approximately the molecular formula KMn₈O₁₆ having a 2x2 hollandite structure. "K-OMS-2" means that the pores (tunnels) of the OMS-2 are occupied by K⁺ ions, which neutralize the negative charge of the OMS-2 framework, consisting of edge- and corner-shared [MnO6]-octahedra.

In a process of the present invention for the production of HMFCA, FFCA and FDCA a base, e.g. a hydroxide, a carbonate or a bicarbonate, e.g. an alkali hydroxide, alkali carbonate or alkali bicarbonate, such as sodium hydroxide, sodium carbonate or sodium bicarbonate may be used as a co-catalyst as well as for increasing the solubility.

In a process of the present invention for the selective production of 2,5-furandicarboxylic acid starting from 5-hydroxymethyl-2-furfural, the combination of the following features has been found to be of particular advantage:
- a base selected from the group of carbonates and bicarbonates, in particular sodium carbonate and/or sodium bicarbonate is used as a co-catalyst
- the working pressure is from 80 to 100 bar.

This embodiment is especially preferred in case the oxidation agent is compressed oxygen. Especially, it has been found that in case of pressures lower than 80 bar deactivation of the catalysts employed was observed, leading to loss of yield in FDCA and loss of selectivity.

The preferred temperature in this embodiment of the present invention is from 120°C to 160°C, more preferably from 140°C to 160°C.

Further preferred, platinum on activated charcoal is used as the catalyst in this embodiment of the present invention.

Again, also in this embodiment, preferably water is used as a solvent. Furthermore, preferably a dipolar aprotic solvent, in particular NMP, is used as a co-solvent.

In contrast to known processes, the present invention provides a single process to synthesize four different furan derivatives of HMF using the same reactor setup just varying reaction parameters such as temperature, pressure, oxidation agent and / or catalyst. This reflects huge benefits in process optimization time, process costs and overall process efficiency impossible to achieve in batch chemistry.

Differently to existing batch protocols in which the reaction conditions need to be optimized from scratch, adapting reaction vessels to the chosen chemistry, the continuous-flow approach avoids these drawbacks in an elegant way. The most significant advantage of the developed process is the reduction of actual reaction volumes to very small volumes (usually lower than 1 mL), which also reduces the safety hazard by orders of magnitude. Even high pressures of pure oxygen can be safely handled and scaled as well - preferably by parallelization of continuous flow reactors rather than increasing reaction volumes.

In the following Reaction Scheme 1 oxidation reactions starting from HMF to obtain the four furan derivatives 5-hydroxymethylfuran-2-carboxylic acid (HMFCA), 2,5-diformylfuran (DFF), 5-formylfuran-2-carboxylic acid (FFCA) and 2,5-furandicarboxylic acid (FDCA) selectively in continuous flow according to the present invention are schematically outlined.

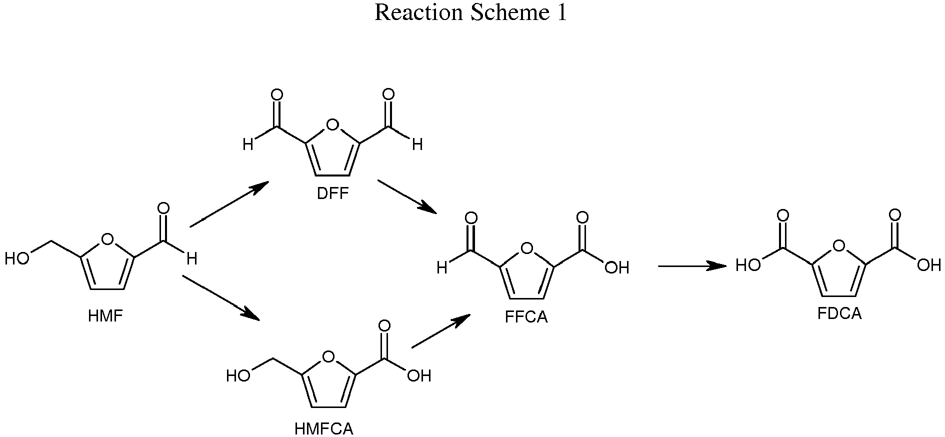

In the following examples all temperatures are in degrees Celsius (°C),

The following abbreviations are used

| | |
|---|---|
| DFF | 2,5-diformylfuran |
| FDCA | 2,5-furandicarboxylic acid |
| FFCA | 5-formylfuran-2-carboxylic acid |
| HMF | 5-hydroxymethyl-2-furfural |
| HMFCA | 5-hydroxymethylfuran-2-carboxylic acid |
| HPLC | high-performance (formerly high-pressure) liquid chromatography |
| K-OMS-2 | manganese octahedral molecular sieve |
| min | minutes |
| NMP | N-methyl-2-pyrrolidone |
| PDA | photo diode array |
| RI | refractive index |
| T | temperature |
| TFA | trifluoroacetic acid |

The yields in % in the Tables below are calculated based on the amount of the starting material HMF.

The reaction performance was evaluated in terms of HMF conversion and HMFCA, DFF, FFCA or FDCA yield/selectivity using HPLC (column: Phenomenex Rezex RHM 150x7.8mm, mobile phase: 0.1 wt% TFA in H₂O, temperature: 85°C, flow rate: 0.6 mL/min, method duration: 23 min (NMP-free samples) / 60 min (NMP-containing samples), detection: RI or PDA, internal standard: phenol).

### Example 1

### Oxidation of HMF to obtain HMFCA

| | |
|---|---|
| Reactant | HMF (5 mg/mL) in water |
| Base additive | NaOH (2 equiv. based on HMF, mixed in situ with the solution of HMF via the second HPLC pump, supplied as 0.08 M solution in water) |
| Catalyst | 10% Pt/C (280 mg 10% Pt/C + 20 mg Celite 545) |
| Oxidant | synthetic air |
| Reactor System | ThalesNano X-Cube, pump flow rate: 2x0.5 mL/min, residence time: 1 min |

Each CatCart (70x4 mm) was filled first with 20 mg of Celite 545 and then 280 mg 10% Pt/C were added. Fresh CatCart was used every time, when the system pressure was changed. Before each screening series, the entire reaction line was purged with H₂O (HPLC Grade), the Teflon frit of the system valve was replaced and ThalesNano X-Cube System Self-Test was performed. The initial system stabilization was always achieved using NaOH / H₂O solution and when the reaction parameters remained constant, the pumping of the reaction solution began, then the system was allowed to stabilize and equilibrate at the new conditions for 10 min and two samples of 1 mL each were then collected. Then the temperature was increased and the system was again allowed to stabilize (the same procedure was applied for all temperatures within the experimental series). In all the cases 40 bar difference between the system pressure and the external gas pressure was provided for good system stability. In the selective oxidation of HMF to HMFCA, temperature-mediated catalyst deactivation was used to synthesize HMFCA in favour of the fully oxidized FDCA.

Table 1 below provides a summary of the results from HMF-HMFCA oxidation screening in flow using the following parameters: 0.5 mL HMF (5 mg/mL), 0.5 mL NaOH (0.08 M), H₂O, 10% Pt/C, 80 bar Air, 60-120°C, 0.5 mL/min x 0.5 mL/min, 1 min.

**Table 1**

| **T [°C]** | **HMF conversion [%]** | **DFF yield [%]** | **HMFCA yield [%]** | **FFCA yield [%]** | **FDCA yield [%]** | **FDCA/ HMFCA selectivity [%]** |
|---|---|---|---|---|---|---|
| 60 | 99.71 | 0.32 | 29.49 | 0.91 | 73.73 | 73.94/ 29.58 |
| 80 | 99.71 | 0.32 | 30.66 | 1.26 | 66.39 | 66.58/ 30.75 |
| 100 | 99.46 | 0.58 | 32.92 | 0.62 | 58.07 | 58.37/ 33.10 |
| 120 | 95.57 | 0.32 | 80.65 | 3.07 | 20.15 | 21.09/ 84.39 |

From Table 1 it is evident that with increasing temperature the HMFCA yield is increasing under the given conditions. The reaction preferably is carried out from 60°C to 120°C, in particular from 80°C to 120°C, in particular from 100 to 120°C. A sharp increase in HMFCA yield is obtained if the temperature exceeds 100°C. A particular preferred temperature is thus from 105 to 130°C, such as 110 to 125°C, e.g. 115 to 120°C.

### Example 2

### Oxidation of HMF to obtain DFF

| | |
|---|---|
| Reactant | HMF (5 mg/mL) in water |
| Catalyst | K-OMS-2 (263.4 mg K-OMS-2 + 50 mg Celite 545) prepared according to Angew. Chem. Int. Ed. 2012, 51, 544-547. |
| Oxidant | oxygen or synthetic air |
| Reactor System | ThalesNano X-Cube, pump flow rate: 0.5 mL/min, residence time: 2/4 min |

Each CatCart (70x4 mm) was filled first with 50 mg Celite 545 and then 263.4 mg K-OMS-2 were added. Fresh CatCart was used every time, when the system pressure was changed. Before each screening series, the entire reaction line was purged with H₂O (HPLC Grade), the Teflon frit of the system valve was replaced and ThalesNano X-Cube System Self-Test was performed. The initial system stabilization was always achieved using H₂O (HPLC Grade) and when the reaction parameters remained constant, the pumping of the reaction solution began, then the system was allowed to stabilize and equilibrate at the new conditions for 10 min and two samples of 1 mL each were then collected. Then the temperature was increased and the system was again allowed to stabilize (the same procedure was applied for all temperatures within the experimental series). In all the cases 40 bar difference between the system pressure and the external gas pressure was provided for good system stability.

The experiments were carried out using one or two catalyst cartridges offering ideal reaction conditions to produce DFF in good yield (∼70%) requiring only 10 bar of oxygen partial pressure.

To reduce the hazardous potential of pure oxygen, the reactions were also performed substituting oxygen with synthetic air. However, to reach similar yields, the pressure had to be increased to 80 bar of compressed air.

In Table 2 below there is set out a summary of the results from HMF-DFF oxidation screening in flow using the following parameters:
1 mL HMF (5 mg/mL), H₂O, K-OMS-2/Celite, 10 bar O₂, 100-160°C, 0.5 mL/min, 2 min (using one catalyst cartridge).

**Table 2**

| **T [°C]** | **HMF conversion [%]** | **DFF yield [%]** | **DFF selectivity [%]** | **HMFCA yield [%]** | **FFCA yield [%]** | **FDCA yield [%]** |
|---|---|---|---|---|---|---|
| 100°C | 30.97 | 20.24 | 65.47 | 0.00 | 4.63 | 0.15 |
| 110°C | 40.80 | 28.51 | 70.19 | 0.00 | 3.01 | 0.00 |
| 120°C | 49.97 | 37.13 | 74.51 | 0.00 | 4.77 | 0.00 |
| 130°C | 61.42 | 48.43 | 79.06 | 0.00 | 7.44 | 0.00 |
| 140°C | 73.19 | 54.23 | 74.08 | 0.00 | 10.09 | 0.00 |
| 150°C | 82.76 | 63.16 | 76.32 | 0.00 | 12.83 | 0.26 |
| 160°C | 88.74 | 69.00 | 77.88 | 0.00 | 14.55 | 0.89 |

In Table 3 below there is set out a summary of the results from HMF-DFF oxidation screening in flow using the following parameters:
1 mL HMF (5 mg/mL), H₂O, 2x K-OMS-2/Celite, 10 bar O₂, 100-160°C, 0.5 mL/min, 4 min (using two catalyst cartridges)

**Table 3**

| **T [°C]** | **HMF conversion [%]** | **DFF yield [%]** | **DFF selectivity [%]** | **HMFCA yield [%]** | **FFCA yield [%]** | **FDCA yield [%]** |
|---|---|---|---|---|---|---|
| 100 | 47.91 | 35.48 | 74.09 | 0.00 | 12.78 | 1.82 |
| 110 | 60.07 | 47.51 | 79.10 | 0.00 | 9.66 | 0.00 |
| 120 | 72.07 | 57.78 | 80.28 | 0.00 | 13.84 | 0.00 |
| 130 | 84.74 | 61.49 | 72.56 | 0.00 | 19.96 | 0.47 |
| 140 | 90.40 | 67.15 | 74.28 | 0.00 | 21.76 | 1.92 |
| 150 | 96.80 | 62.45 | 64.52 | 0.00 | 28.42 | 3.54 |
| 160 | 98.74 | 59.00 | 59.76 | 0.00 | 28.97 | 6.09 |

In Table 4 below there is set out a summary of the results from HMF-DFF oxidation screening in flow using the following parameters:
1 mL HMF (5 mg/mL), H₂O, K-OMS-2/Celite, 80 bar Air, 100-160°C, 0.5 mL/min, 2 min (using one catalyst cartridge).

**Table 4**

| **T [°C]** | **HMF conversion [%]** | **DFF yield [%]** | **DFF selectivity [%]** | **HMFCA yield [%]** | **FFCA yield [%]** | **FDCA yield [%]** |
|---|---|---|---|---|---|---|
| 100 | 32.31 | 19.17 | 59.33 | 0.00 | 1.04 | 0.00 |
| 110 | 42.28 | 30.14 | 71.28 | 0.00 | 2.37 | 0.00 |
| 120 | 54.06 | 39.36 | 72.81 | 0.00 | 4.26 | 0.00 |
| 130 | 68.06 | 48.24 | 70.87 | 0.00 | 7.39 | 0.00 |
| 140 | 78.14 | 57.02 | 72.98 | 0.00 | 9.70 | 0.00 |
| 150 | 82.29 | 61.83 | 75.14 | 0.00 | 9.06 | 0.00 |
| 160 | 84.97 | 63.69 | 74.96 | 0.00 | 10.52 | 0.00 |

In Table 5 below there is set out a summary of the results from HMF-DFF oxidation, screening in flow using the following parameters:
1 mL HMF (5 mg/mL), H₂O, 2x K-OMS-2/Celite, 80 bar Air, 100-160°C, 0.5 mL/min, 4 min (using two catalyst cartridge).

**Table 5**

| **T [°C]** | **HMF conversion [%]** | **DFF yield [%]** | **DFF selectivity [%]** | **HMFCA yield [%]** | **FFCA yield [%]** | **FDCA yield [%]** |
|---|---|---|---|---|---|---|
| 100 | 60.53 | 36.24 | 60.30 | 0.00 | 19.86 | 0.00 |
| 110 | 64.16 | 44.51 | 69.39 | 0.00 | 10.01 | 0.00 |
| 120 | 76.13 | 52.80 | 69.37 | 0.00 | 12.93 | 0.00 |
| 130 | 85.77 | 59.16 | 68.97 | 0.00 | 16.53 | 0.00 |
| 140 | 92.83 | 61.12 | 65.85 | 0.00 | 20.26 | 0.00 |
| 150 | 95.93 | 65.46 | 68.24 | 0.00 | 19.95 | 0.00 |
| 160 | 95.18 | 66.61 | 69.98 | 0.00 | 17.45 | 0.00 |

From Tables 2 to 5 it is evident that under the given conditions a high DFF yields and a high DFF selectivity may be achieved. The yield in average is increasing with increasing temperature. A double portion of the catalyst does not result in great differences, nor does a pressure of 80 bar compared with a pressure of 10 bar.

A temperature yielding DFF in a range of approx. 50 to 70% related to the starting material HMF is in the range from approx. 100 to 160°C, e.g. 120°C to 160°C, e.g. 140 to 160°C.

### Example 3

### Oxidation of HMF to obtain FFCA

| | |
|---|---|
| Reactant | HMF (5 mg/mL) in water |
| Base additive | Na₂CO₃ (2 equiv. based on HMF, premixed with HMF solution) |
| Catalyst | 10% Pt/C (280 mg 10% Pt/C + 20 mg Celite 545) |
| Oxidant | synthetic air |
| Reactor System | ThalesNano X-Cube, pump flow rate: 0.5mL/min, residence time: 2 min |

Each CatCart (70x4 mm) was filled first with 20 mg Celite 545 and then 280 mg 10% Pt/C were added. Fresh CatCart was used every time, when the system pressure was changed. Before each screening series, the entire reaction line was purged with H₂O (HPLC Grade), the Teflon frit of the system valve was replaced and ThalesNano X-Cube System Self-Test was performed. The initial system stabilization was always achieved using H₂O (HPLC Grade) and when the reaction parameters remained constant, the pumping of the reaction solution began, then the system was allowed to stabilize and equilibrate at the new conditions for 10 min and two samples of 1 mL each were then collected. Then the temperature was increased and the system was again allowed to stabilize (the same procedure was applied for all temperatures within the experimental series). In all the cases 40 bar difference between the system pressure and the external gas pressure was provided for good system stability. At a temperature of 100°C, an ideal compromise between substrate conversion and product selectivity regarding the product FFCA was achieved.

In Table 6 below there is set out a summary of the results from HMF-FFCA oxidation screening in flow using the following parameters:
1 mL HMF (5 mg/mL), 2 equiv. Na₂CO₃, H₂O, 10% Pt/C, 80 bar Air, 60-160°C, 0.5 mL/min, 2 min.

**Table 6**

| **T [°C]** | **HMF conversion [%]** | **DFF yield [%]** | **HMFCA yield [%]** | **FFCA yield [%]** | **FDCA yield [%]** | **FDCA/ FFCA selectivity [%]** |
|---|---|---|---|---|---|---|
| 60 | 99.71 | 0.32 | 0.00 | 0.00 | 57.70 | 57.87/ 0.00 |
| 80 | 99.71 | 0.32 | 1.84 | 44.90 | 43.28 | 43.41/ 45.03 |
| 100 | 98.61 | 0.32 | 5.90 | 60.26 | 25.42 | 25.78/ 61.11 |
| 120 | 94.15 | 0.32 | 6.55 | 56.77 | 19.80 | 21.03/ 60.30 |
| 140 | 92.01 | 0.32 | 7.44 | 48.01 | 15.69 | 17.06/ 52.18 |
| 160 | 85.69 | 0.32 | 9.87 | 25.41 | 13.44 | 15.69/ 29.65 |

From Table 6 it is evident that under the given conditions a high FFCA yield and a high FFCA selectivity may be achieved. The yield in average is increasing with increasing temperature up to approx. 120°C. A temperature yielding FFCA in a range of approx. 45 to 60% related to the starting material HMF is in the range from 60°C to 160°C, in particular from 80 to 140°C, e.g. 100 to 120°C.

### Example 4

### Oxidation of HMF to obtain FDCA

| | |
|---|---|
| Reactant | HMF (5 mg/mL) in water |
| Base additive | NaOH (2 equiv. based on HMF, mixed in situ with the solution of HMF via the second HPLC pump, supplied as 0.08 M solution in water) or Na₂CO₃ (2 equiv. based on HMF, premixed with HMF solution) or NaHCO₃ (4 equiv. based on HMF, premixed with HMF solution) |
| Catalyst | 10% Pt/C (280 mg 10% Pt/C + 20 mg Celite 545) |
| Oxidant | oxygen or synthetic air |
| Reactor System | ThalesNano X-Cube, pump flow rate: 2x0.5 mL/min (NaOH), 0.5 mL/min (Na₂CO₃), 0.5 mL/min (NaHCO₃), residence time: 1 min (NaOH), 2 min (Na₂CO₃), 2 min (NaHCO₃) |

Each CatCart (70x4 mm) was filled first with 20 mg of Celite 545 and then 280 mg of 10% Pt/C were added. Fresh CatCart was used every time, when the system pressure was changed. Before each screening series, the entire reaction line was purged with H₂O (HPLC Grade), the Teflon frit of the system valve was replaced and ThalesNano X-Cube System Self-Test was performed. The initial system stabilization was always achieved using either NaOH / H₂O solution, or H₂O (HPLC grade). Using either Na₂CO₃ or NaHCO₃ as base additive, the system was stabilized while pumping only H₂O (HPLC grade), not Na₂CO₃ or NaHCO₃ aqueous solution. When the reaction parameters remained constant, the pumping of the reaction solution began, then the system was allowed to stabilize and equilibrate at the new conditions for 10 min and two samples of 1 mL each were then collected. Then the temperature was increased and the system was again allowed to stabilize (the same procedure was applied for all temperatures within the experimental series). In all the cases 40 bar difference between the system pressure and the external gas pressure was provided for good system stability.

Initial experiments were carried out using NaOH as a base. Unfortunately, treating HMF solution with NaOH solution led to immediate dark colouring of the solution, followed by precipitation of black solid material rendering the solution inapplicable in flow. To overcome this problem, in-situ mixing of HMF solution and NaOH solution was performed. However, even better results were obtained switching from NaOH solution to Na₂CO₃ or NaHCO₃ solution.

In Table 7 below there is set out a summary of the results from HMF-FDCA oxidation screening in flow using the following parameters:
0.5 mL HMF (5 mg/mL), 0.5 mL NaOH (0.08 M), H₂O, 10% Pt/C, 40 bar O₂, 60-160°C, 0.5 mL/min x 0.5 mL/min, 1 min.

**Table 7**

| **T [°C]** | **HMF conversion [%]** | **DFF yield [%]** | **HMFCA yield [%]** | **FFCA yield [%]** | **FDCA yield [%]** | **FDCA selectivity [%]** |
|---|---|---|---|---|---|---|
| 60 | 99.71 | 0.32 | 18.97 | 6.78 | 70.98 | 71.19 |
| 80 | 99.71 | 0.32 | 14.18 | 10.48 | 77.23 | 77.46 |
| 100 | 99.64 | 0.67 | 7.30 | 18.60 | 79.41 | 79.70 |
| 120 | 99.50 | 0.81 | 2.08 | 22.28 | 78.76 | 79.16 |
| 140 | 99.43 | 0.32 | 0.36 | 25.16 | 74.14 | 74.57 |
| 160 | 99.71 | 0.32 | 23.95 | 1.28 | 68.87 | 69.07 |

In Table 8 below there is set out a summary of the results from HMF-FDCA oxidation screening in flow using the following parameters:
0.5 mL HMF (5 mg/mL), 0.5 mL NaOH (0.08 M), H₂O, 10% Pt/C, 80 bar O₂, 60-160°C, 0.5 mL/min x 0.5 mL/min, 1 min.

**Table 8**

| **T [°C]** | **HMF conversion [%]** | **DFF yield [%]** | **HMFCA yield [%]** | **FFCA yield [%]** | **FDCA yield [%]** | **FDCA selectivity [%]** |
|---|---|---|---|---|---|---|
| 60 | 99.71 | 0.32 | 17.23 | 8.20 | 74.15 | 74.37 |
| 80 | 99.71 | 0.32 | 11.79 | 10.67 | 79.33 | 79.57 |
| 100 | 99.59 | 0.81 | 6.16 | 17.31 | 77.97 | 78.29 |
| 120 | 99.06 | 0.32 | 1.68 | 23.86 | 76.25 | 76.98 |
| 140 | 99.34 | 0.32 | 0.79 | 31.01 | 64.85 | 65.28 |
| 160 | 99.71 | 0.32 | 27.81 | 1.29 | 57.83 | 58.00 |

From Tables 7 and 8 it is evident that under the given conditions a high FDCA yield and a high FDCA selectivity may be achieved almost independently from the temperature. A temperature yielding FDCA in a range of approx. 60 to 80% related to the starting material HMF is in the range from 60 to 160°C, e.g. 80 to 150°C.

In Table 9 below there is set out a summary of the results from HMF-FDCA oxidation screening in flow using the following parameters:
0.5 mL HMF (5 mg/mL), 0.5 mL NaOH (0.08 M), H₂O, 10% Pt/C, 40 bar Air, 60-120°C, 0.5 mL/min x 0.5 mL/min, 1 min.

**Table 9**

| **T [°C]** | **HMF conversion [%]** | **DFF yield [%]** | **HMFCA yield [%]** | **FFCA yield [%]** | **FDCA yield [%]** | **FDCA selectivity [%]** |
|---|---|---|---|---|---|---|
| 60 | 99.71 | 0.32 | 3.66 | 14.45 | 76.84 | 77.06 |
| 80 | 99.71 | 0.32 | 8.59 | 25.31 | 65.02 | 65.21 |
| 100 | 98.00 | 0.32 | 7.12 | 27.27 | 54.09 | 55.19 |
| 120 | 83.86 | 0.32 | 13.28 | 24.25 | 31.09 | 37.06 |

From Table 9 it is evident that under the given conditions a high FDCA yield and a high FDCA selectivity may be achieved. A temperature yielding FDCA in a range of approx. 60 to 80% related to the starting material HMF is in the range from 60 to 120°C, e.g. 60 to 110°C.

In Table 10 below there is set out a summary of the results from HMF-FDCA oxidation screening in flow using the following parameters:
1 mL HMF (5 mg/mL), 2 equiv. Na₂CO₃, H₂O, 10% Pt/C, 80 bar O₂, 60-120°C, 0.5 mL/min, 2 min.

**Table 10**

| **T [°C]** | **HMF conversion [%]** | **DFF yield [%]** | **HMFCA yield [%]** | **FFCA yield [%]** | **FDCA yield [%]** | **FDCA selectivity [%]** |
|---|---|---|---|---|---|---|
| 60 | 99.71 | 0.32 | 0.00 | 0.00 | 79.02 | 79.25 |
| 80 | 99.68 | 0.32 | 0.00 | 0.00 | 91.44 | 91.73 |
| 100 | 99.71 | 0.32 | 0.00 | 0.00 | 95.23 | 95.51 |
| 120 | 99.71 | 0.32 | 0.00 | 0.00 | 95.23 | 95.51 |

From Table 10 it is evident that a high conversion rate of HMF and high yields of FDCA with high selectivity can be achieved from approx. 50°C to 140°C under the given conditions, and an almost complete conversion of HMF into FDCA in a temperature range of approx. 70 to 130°C.

Carrying out the example with the same reaction setup, with the only difference in that O₂-pressure was reduced to 40 bar, the following results were achieved:

**Table 11**

| **T [°C]** | **HMF conversion [%]** | **DFF yield [%]** | **HMFCA yield [%]** | **FFCA yield [%]** | **FDCA yield [%]** | **FDCA selectivity [%]** |
|---|---|---|---|---|---|---|
| 60 | 99.55 | 0.32 | 0.00 | 0.00 | 78.62 | 78.97 |
| 80 | 99.55 | 0.32 | 11.45 | 0.00 | 68.62 | 68.94 |
| 100 | 99.51 | 0.32 | 13.96 | 0.00 | 58.15 | 58.43 |
| 120 | 99.42 | 0.32 | 12.20 | 0.00 | 49.36 | 49.65 |

Table 11 shows that with lower oxygen pressure, both FDCA yield and selectivity are decreased especially with higher temperature. This is apparently due to catalyst deactivation.

In Table 12 below there is set out a summary of the results from HMF-FDCA oxidation screening in flow using the following parameters:
1 mL HMF (5 mg/mL), 4 equiv. NaHCO₃, H₂O, 10% Pt/C, 80 bar O₂, 60-120°C, 0.5 mL/min, 2 min.

**Table 12**

| **T [°C]** | **HMF conversion [%]** | **DFF yield [%]** | **HMFCA yield [%]** | **FFCA yield [%]** | **FDCA yield [%]** | **FDCA selectivity [%]** |
|---|---|---|---|---|---|---|
| 60 | 99.71 | 0.32 | 0.00 | 0.00 | 73.46 | 73.67 |
| 80 | 99.71 | 0.32 | 0.00 | 7.51 | 87.82 | 88.08 |
| 100 | 99.71 | 0.32 | 0.00 | 2.07 | 90.33 | 90.59 |
| 120 | 99.71 | 0.32 | 0.00 | 0.00 | 96.46 | 96.74 |

From Table 12 it is evident that a high conversion rate of HMF and high yields of FDCA with high selectivity can be achieved from approx. 50°C to 140°C under the given conditions, and an almost complete conversion of HMF into FDCA at temperatures above 100°C, e.g. of approx. 110°C to 130°C.

Again, carrying out this example with the same reaction setup, with the only difference in that O₂-pressure was reduced to 40 bar, the following results were achieved:

**Table 13**

| **T [°C]** | **HMF conversion [%]** | **DFF yield [%]** | **HMFCA yield [%]** | **FFCA yield [%]** | **FDCA yield [%]** | **FDCA selectivity [%]** |
|---|---|---|---|---|---|---|
| 60 | 99.55 | 0.32 | 0.00 | 0.00 | 66.74 | 67.04 |
| 80 | 99.55 | 0.32 | 0.00 | 5.95 | 84.79 | 85.17 |
| 100 | 99.55 | 0.32 | 0.00 | 0.00 | 82.56 | 82.94 |
| 120 | 99.55 | 0.32 | 0.00 | 0.00 | 32.14 | 32.29 |

Again, according to Table 13, with lower oxygen pressure, both FDCA yield and selectivity are decreased especially with higher temperature due to catalyst deactivation.

Thus, the above examples show that especially HMF oxidation to FDCA, employing alkali carbonates or bicarbonates as co-catalyst and employing higher oxygen pressure, yields very good results at only 2 minutes of residence time.

### Example 5

### Oxidation of HMF to obtain FDCA employing water as a solvent and NMP as co-solvent:

In this example, an artificial stream enriched with HMF, resembling a stream resulting from a previous dehydration of a sugar, was used as the starting material.

| | |
|---|---|
| Artificial stream solution: | 5 mg/mL HMF, |
| | ratio of HMF : NMP = 4.7 wt% : 95.3 wt% |
| Base additive: | NaHCO₃, 4 equiv based on HMF |
| Solvent: | H₂O added to the artificial stream solution up to 1 mL, the NMP of the artificial stream solution acting as co-solvent |
| Catalyst: | 10% Pt/C / Celite 545 (280 mg / 20 mg) |
| Oxidant: | O₂, pressure: 80 bar |
| Temperature: | 60°C, 80°C, 100°C, 120°C, 140°C, 160°C |
| Flow rate: | 0.5 mL/min |
| Residence time: | 2 min |

The reaction was carried out in accordance with the description of Example 4 above.

In Table 14 below there is set out a summary of the results from HMF-FDCA oxidation screening in flow using the following parameters:
1 mL HMF (5 mg/mL), 4 equiv. NaHCO₃, H₂O/NMP, 10% Pt/C, 80 bar O₂, 60-160°C, 0.5 mL/min, 2 min.

**Table 14**

| **T [°C]** | **HMF conversion [%]** | **DFF yield [%]** | **HMFCA yield [%]** | **FFCA yield [%]** | **FDCA yield [%]** | **FDCA selectivity [%]** |
|---|---|---|---|---|---|---|
| 60 | 99.55 | 0.32 | 2.25 | 38.75 | 43.52 | 43.72 |
| 80 | 99.55 | 0.32 | 0.49 | 39.55 | 59.17 | 59.43 |
| 100 | 99.15 | 0.32 | 0.00 | 19.03 | 77.42 | 78.09 |
| 120 | 99.55 | 0.32 | 0.00 | 6.19 | 90.53 | 90.94 |
| 140 | 99.55 | 0.32 | 0.00 | 1.61 | 92.13 | 92.55 |
| 160 | 99.55 | 0.32 | 0.00 | 0.72 | 80.72 | 81.09 |

From Table 14 above it becomes apparent that also based on a product stream containing NMP, good results in FDCA yield and FDCA selectivity can be obtained. The best results however, are obtained at slightly higher temperatures, such as 120°C to 160°C.

### Example 6

### Oxidation of HMF to obtain FDCA from a raw product stream of a preceding sugar dehydration step:

A product stream obtained via dehydration of fructose with NMP as solvent, as disclosed in WO 2014/033289, was treated under the same conditions as disclosed in example 5 above. Again, the ratio of HMF to NMP in this product stream was
HMF : NMP = 4.7 wt% : 95.3 wt%.

This raw stream was pretreated before oxidation as follows:
(i) real stream dilution with pure water to the desired HMF concentration of 5 mg/mL;
(ii) centrifugation in order to separate any black tar formed during the preparation of the stream;
(iii) filtration through a filter paper;
(iv) passing the resulting solution through a packed-bed cartridge filled with activated charcoal.

In Table 15 below there is set out a summary of the results from HMF-FDCA oxidation screening in flow using the following parameters:
1 mL HMF (5 mg/mL), 4 equiv. NaHCO₃, H₂O/NMP, 10% Pt/C, 80 bar O₂, 60-160°C, 0.5 mL/min, 2 min.

**Table 15**

| **T [°C]** | **HMF conversion [%]** | **DFF yield [%]** | **HMFCA yield [%]** | **FFCA yield [%]** | **FDCA yield [%]** | **FDCA selectivity [%]** |
|---|---|---|---|---|---|---|
| 60 | 99.55 | 0.31 | 6.75 | 50.33 | 10.75 | 10.79 |
| 80 | 98.73 | 0.31 | 6.57 | 72.46 | 10.97 | 11.11 |
| 100 | 98.44 | 0.31 | 4.13 | 71.62 | 18.16 | 18.45 |
| 120 | 98.27 | 0.31 | 1.11 | 61.42 | 35.02 | 35.64 |
| 140 | 98.74 | 0.31 | 0.00 | 32.69 | 64.64 | 65.47 |
| 160 | 99.55 | 0.31 | 0.00 | 8.57 | 87.01 | 87.40 |

Table 15 shows that - although the results are slightly worse than those of an artificial stream as per Example 5 - acceptable results in FDCA yield and selectivity can be obtained, again especially at higher temperatures such as from 140°C to 160°C, without the need of prior removal of NMP from the product stream.

## Claims

1. Process for the selective production of oxidized furan derivatives starting from 5-hydroxymethyl-2-furfural of formula 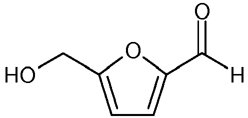 in the presence of a solvent, an oxidation agent, a catalyst, and optionally a base and/or a co-solvent, **characterized in that**
- the oxidation process is carried out continuously in flow,
- the process is carried out in a reactor setup for varying reaction parameters
- the solvent for the oxidation process is water and
- a dipolar aprotic solvent is present as a co-solvent.

2. Process according to claim 1, **characterized in that** N-methylpyrrolidone is present as a co-solvent.

3. Process according to claim 1 or 2, wherein reaction parameters are temperature, pressure, oxidation agent, and/or catalyst.

4. Process according to any one of claims 1 to 3, wherein said oxidized furan derivative comprises at least one aldehyde group and/or at least one carboxylic acid group.

5. Process according to claim 4, wherein said oxidized furan derivative is selected from 5-hydroxymethylfuran-2-carboxylic acid of formula 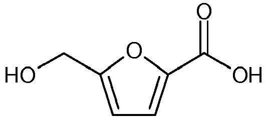 2,5-diformylfuran of formula 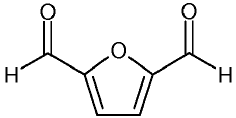 5-formylfuran-2-carboxylic acid of formula 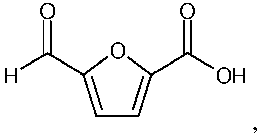 and 2,5-furandicarboxylic acid of formula

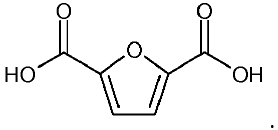

6. Process according to any one of claims 1 to 5, **characterized in that** the reaction temperature is from 50°C to 180°C, in particular from 60°C to 160°C.

7. Process according to claim 6, wherein the reaction temperature for the production of
- 5-hydroxymethylfuran-2-carboxylic acid is from 60°C to 120°C, in particular from 80°C to 120°C, in particular from 100 to 120°C;
- 2,5-diformylfuran is from 100 to 160°C, in particular from 120-160°C, in particular from 140°C to 160°C;
- 5-formylfuran-2-carboxylic acid is from 60°C to 160°C, in particular from 80°C to 140°C, in particular from 100°C to 120°C;
- 2,5-furandicarboxylic acid is from 60°C to 160°C, in particular from 60°C to 120°C, in particular from 80°C to 120°C.

8. Process according to any one of claims 1 to 7, **characterized in that** the oxidation agent is compressed oxygen or compressed air.

9. Process according to any one of claims 1 to 8, **characterized in that** the working pressure is from 5 bar to 100 bar, in particular from 10 bar to 80 bar.

10. Process according to any one of claims 1 to 9, **characterized in that** the catalyst used to obtain
- 2,5-diformylfuran is K-OMS-2;
- 5-hydroxymethylfuran-2-carboxylic acid, 5-formylfuran-2-carboxylic acid and 2,5-furandicarboxylic acid is platinum on activated charcoal.

11. Process according to any one of claims 1 to 10, wherein for the production of 5-hydroxymethylfuran-2-carboxylic acid, 5-formylfuran-2-carboxylic acid and 2,5-furandicarboxylic acid a base is used as a co-catalyst.

12. Process according to any one of claims 1 to 11, **characterized in that** the base is a hydroxide, a carbonate or a bicarbonate, in particular an alkali hydroxide, an alkali carbonate or an alkali bicarbonate, in particular sodium hydroxide, sodium carbonate or sodium bicarbonate.

13. Process according to any one of the preceding claims, **characterized in that** a stream enriched with 5-hydroxymethyl-2-furfural from previous dehydration reactions, in particular dehydrations of sugars, is employed as a starting material.

14. Process according to any one of the preceding claims for the selective production of 2,5-furandicarboxylic acid starting from 5-hydroxymethyl-2-furfural, **characterized by** the combination of the following features:
- a base selected from the group of carbonates and bicarbonates, in particular sodium carbonate and/or sodium bicarbonate is used as a co-catalyst
- the working pressure is from 80 to 100 bar.

15. Process according to claim 14, **characterized in that** the temperature is from 120°C to 160°C, preferably from 140°C to 160°C.

16. Process according to claim 14 and 15, **characterized in that** platinum on activated charcoal is used as the catalyst.

## Patentansprüche

1. Verfahren zur selektiven Herstellung von oxidierten Furanderivaten ausgehend von 5-Hydroxymethyl-2-furfural der Formel 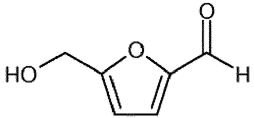 in Gegenwart eines Lösungsmittels, eines Oxidationsmittels, eines Katalysators und gegebenenfalls einer Base und/oder eines Co-Lösungsmittels, **dadurch gekennzeichnet, dass**
- der Oxidationsvorgang kontinuierlich im Fluss erfolgt,
- das Verfahren in einer Reaktoranordnung zur Variation von Reaktionsparametern durchgeführt wird,
- das Lösungsmittel für den Oxidationsvorgang Wasser ist und
- ein dipolares aprotisches Lösungsmittel als Co-Lösungsmittel vorhanden ist.

2. Verfahren nach Anspruch 1, **dadurch gekennzeichnet, dass** N-Methylpyrrolidon als Co-Lösungsmittel vorhanden ist.

3. Verfahren nach Anspruch 1 oder 2, wobei Reaktionsparameter Temperatur, Druck, Oxidationsmittel und/oder Katalysator sind.

4. Verfahren nach einem der Ansprüche 1 bis 3, wobei das oxidierte Furanderivat zumindest eine Aldehydgruppe und/oder zumindest eine Carbonsäuregruppe umfasst.

5. Verfahren nach Anspruch 4, wobei das oxidierte Furanderivat aus 5-Hydroxymethylfuran-2-carbonsäure der Formel 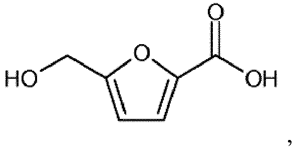 2,5-Diformylfuran der Formel 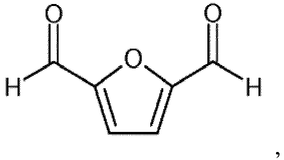 5-Formylfuran-2-carbonsäure der Formel 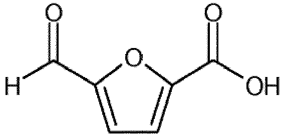 und 2,5-Furandicarbonsäure der Formel 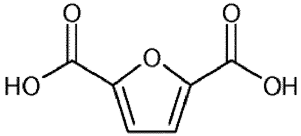 ausgewählt ist.

6. Verfahren nach einem der Ansprüche 1 bis 5, **dadurch gekennzeichnet, dass** die Reaktionstemperatur von 50 °C bis 180 °C, insbesondere von 60 °C bis 160 °C, beträgt.

7. Verfahren nach Anspruch 6, wobei die Reaktionstemperatur für die Herstellung von
- 5-Hydroxymethylfuran-2-carbonsäure von 60 °C bis 120 °C, insbesondere von 80 °C bis 120 °C, insbesondere von 100 °C bis 120 °C, beträgt;
- 2,5-Diformylfuran von 100 °C bis 160 °C, insbesondere von 120 °C bis 160 °C, insbesondere von 140 °C bis 160 °C, beträgt;
- 5-Formylfuran-2-carbonsäure von 60 °C bis 160 °C, insbesondere von 80 °C bis 140 °C, insbesondere von 100 °C bis 120 °C, beträgt;
- 2,5-Furandicarbonsäure von 60 °C bis 160 °C, insbesondere von 60 °C bis 120 °C, insbesondere von 80 °C bis 120 °C, beträgt.

8. Verfahren nach einem der Ansprüche 1 bis 7, **dadurch gekennzeichnet, dass** das Oxidationsmittel komprimierter Sauerstoff oder komprimierte Luft ist.

9. Verfahren nach einem der Ansprüche 1 bis 8, **dadurch gekennzeichnet, dass** der Arbeitsdruck von 5 bar bis 100 bar, insbesondere von 10 bar bis 80 bar, beträgt.

10. Verfahren nach einem der Ansprüche 1 bis 9, **dadurch gekennzeichnet, dass** der Katalysator, der zum Erhalt von
- 2,5-Diformylfuran verwendet wird, K-OMS-2 ist;
- 5-Hydroxymethylfuran-2-carbonsäure, 5-Formylfuran-2-carbonsäure und 2,5-Furandicarbonsäure verwendet wird, Platin auf Aktivkohle ist.

11. Verfahren nach einem der Ansprüche 1 bis 10, wobei für die Herstellung von 5-Hydroxymethylfuran-2-carbonsäure, 5-Formylfuran-2-carbonsäure und 2,5-Furandicarbonsäure eine Base als Cokatalysator verwendet wird.

12. Verfahren nach einem der Ansprüche 1 bis 11, **dadurch gekennzeichnet, dass** die Base ein Hydroxid, ein Carbonat oder ein Bicarbonat ist, insbesondere ein Alkalihydroxid, ein Alkalicarbonat oder ein Alkalibicarbonat, insbesondere Natriumhydroxid, Natriumcarbonat oder Natriumbicarbonat.

13. Verfahren nach einem der vorangegangenen Ansprüche, **dadurch gekennzeichnet, dass** ein mit 5-Hydroxymethyl-2-furafural aus vorangegangenen Dehydratisierungsreaktionen, insbesondere Dehydratisierungen von Zucker, angereicherter Strom als Ausgangsmaterial eingesetzt wird.

14. Verfahren nach einem der vorangegangenen Ansprüche zur selektiven Herstellung von 2,5-Furandicarbonsäure ausgehend von 5-Hydroxymethyl-2-furfural, **gekennzeichnet durch** die Kombination der folgenden Merkmale:
- eine aus der aus Carbonaten und Bicarbonaten bestehenden Gruppe ausgewählte Base, insbesondere Natriumcarbonat und/oder Natriumbicarbonat, wird als Cokatalysator verwendet
- der Arbeitsdruck beträgt von 80 bis 100 bar.

15. Verfahren nach Anspruch 14, **dadurch gekennzeichnet, dass** die Temperatur von 120 °C bis 160 °C, vorzugsweise von 140 °C bis 160 °C, beträgt.

16. Verfahren nach Anspruch 14 und 15, **dadurch gekennzeichnet, dass** Platin auf Aktivkohle als Katalysator verwendet wird.

## Revendications

1. Procédé pour la production sélective de dérivés furanes oxydés à partir de 5-hydroxyméthyl-2-furfural de formule 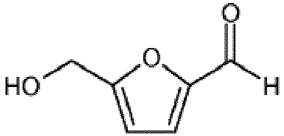 en présence d'un solvant, d'un agent d'oxydation, d'un catalyseur, et éventuellement d'une base et/ou d'un co-solvant, **caractérisé en ce que**
- le procédé d'oxydation est réalisé de manière continue en flux,
- le procédé est réalisé dans un réacteur configuré pour faire varier les paramètres de réaction
- le solvant pour le procédé d'oxydation est l'eau et
- un solvant aprotique dipolaire est présent en tant que co-solvant.

2. Procédé selon la revendication 1, **caractérisé en ce que** de la N-méthylpyrrolidone est présente en tant que co-solvant.

3. Procédé selon la revendication 1 ou 2, dans lequel les paramètres de réaction sont la température, la pression, l'agent d'oxydation et/ou le catalyseur.

4. Procédé selon l'une quelconque des revendications 1 à 3, dans lequel ledit dérivé furane oxydé comprend au moins un groupe aldéhyde et/ou au moins un groupe acide carboxylique.

5. Procédé selon la revendication 4, dans lequel ledit dérivé furane oxydé est choisi parmi l'acide 5-hydroxyméthylfuran-2-carboxylique de formule 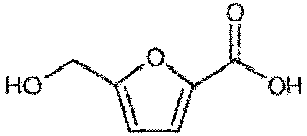 le 2,5-diformylfurane de formule 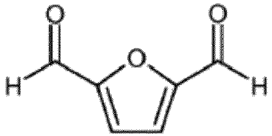 l'acide 5-formylfuran-2-carboxylique de formule 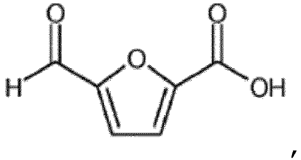 et l'acide 2,5-furanedicarboxylique de formule

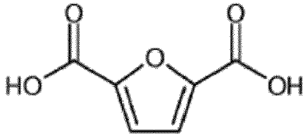

6. Procédé selon l'une quelconque des revendications 1 à 5, **caractérisé en ce que** la température de réaction est de 50 °C à 180 °C, en particulier de 60 °C à 160 °C.

7. Procédé selon la revendication 6, dans lequel la température de réaction pour la production de
- acide 5-hydroxyméthylfuran-2-carboxylique est de 60 °C à 120 °C, en particulier de 80 °C à 120 °C, en particulier de 100 à 120 °C ;
- 2,5-diformylfurane est de 100 à 160 °C, en particulier de 120 à 160 °C, en particulier de 140 °C à 160 °C ;
- acide 5-formylfuran-2-carboxylique est de 60 °C à 160 °C, en particulier de 80 °C à 140 °C, en particulier de 100 °C à 120 °C ;
- acide 2,5-furanedicarboxylique est de 60 °C à 160 °C, en particulier de 60 °C à 120 °C, en particulier de 80 °C à 120 °C.

8. Procédé selon l'une quelconque des revendications 1 à 7, **caractérisé en ce que** l'agent d'oxydation est de l'oxygène comprimé ou de l'air comprimé.

9. Procédé selon l'une quelconque des revendications 1 à 8, **caractérisé en ce que** la pression de travail est de 5 bars à 100 bars, en particulier de 10 bars à 80 bars.

10. Procédé selon l'une quelconque des revendications 1 à 9, **caractérisé en ce que** le catalyseur utilisé pour obtenir
- le 2,5-diformylfurane est K-OMS-2 ;
- l'acide 5-hydroxyméthylfuran-2-carboxylique, l'acide 5-formylfuran-2-carboxylique et l'acide 2,5-furanedicarboxylique est le platine sur charbon activé.

11. Procédé selon l'une quelconque des revendications 1 à 10, dans lequel pour la production de l'acide 5-hydroxyméthylfuran-2-carboxylique, de l'acide 5-formylfuran-2-carboxylique et de l'acide 2,5-furanedicarboxylique, une base est utilisée en tant que co-catalyseur.

12. Procédé selon l'une quelconque des revendications 1 à 11, **caractérisé en ce que** la base est un hydroxyde, un carbonate ou un bicarbonate, en particulier un hydroxyde alcalin, un carbonate alcalin ou un bicarbonate alcalin, en particulier l'hydroxyde de sodium, le carbonate de sodium ou le bicarbonate de sodium.

13. Procédé selon l'une quelconque des revendications précédentes, **caractérisé en ce qu'**un courant enrichi en 5-hydroxyméthyl-2-furfural provenant de réactions de déshydratation antérieures, en particulier de déshydratations de sucres, est utilisé comme matériau de départ.

14. Procédé selon l'une quelconque des revendications précédentes pour la production sélective d'acide 2,5-furanedicarboxylique à partir de 5-hydroxyméthyl-2-furfural, **caractérisé par** la combinaison des caractéristiques suivantes :
- une base choisie dans le groupe des carbonates et des bicarbonates, en particulier le carbonate de sodium et/ou le bicarbonate de sodium est utilisée en tant que co-catalyseur
- la pression de travail est de 80 à 100 bars.

15. Procédé selon la revendication 14, **caractérisé en ce que** la température est de 120 °C à 160 °C, de préférence de 140 °C à 160 °C.

16. Procédé selon les revendications 14 et 15, **caractérisé en ce que** du platine sur charbon activé est utilisé en tant que catalyseur.
